(19) Europäisches Patentamt / European Patent Office / Office européen des brevets

(11) **EP 2 374 485 A1**

(12) **EUROPEAN PATENT APPLICATION**

(43) Date of publication:
**12.10.2011 Bulletin 2011/41**

(51) Int Cl.:
*A61L 27/60* (2006.01)     *A61Q 19/00* (2006.01)
*A61L 27/38* (2006.01)

(21) Application number: **11425098.8**

(22) Date of filing: **11.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.04.2010 IT MI20100601**

(71) Applicants:
- **Benassi, Luisa**
  **41051 Montale Rangone (MO) (IT)**
- **Giannetti, Alberto**
  **27100 Pavia (IT)**
- **Magnoni, Cristina**
  **41019 Soliera (MO) (IT)**

- **Pellegrini, Massimo**
  **42122 Reggio Emilia (IT)**

(72) Inventors:
- **Benassi, Luisa**
  **41051 Montale Rangone (MO) (IT)**
- **Giannetti, Alberto**
  **27100 Pavia (IT)**
- **Magnoni, Cristina**
  **41019 Soliera (MO) (IT)**
- **Pellegrini, Massimo**
  **42122 Reggio Emilia (IT)**

(74) Representative: **Modiano, Micaela Nadia et al**
**Dr. Modiano & Associati SpA**
**Via Meravigli 16**
**20123 Milano (IT)**

(54) **New dermal substitute and therapeutical application thereof**

(57)     The subject-matter of the present invention is a new dermal substitute, comprising adipose tissue-derived mesenchymal stem cells and optionally precursors of adipose tissue-derived endothelial cells (VECs), inserted in a biocompatible matrix, and its use to facilitate wound repairing and skin regeneration.

⊠ scaffold

⊙ ASDC

Fig. 4

EP 2 374 485 A1

**Description**

The present invention relates to a new dermal substitute and to its use in order to facilitate the repairing of wounds and skin regeneration.

The repairing of wounds is a coordinated and complex process that involves fundamental biochemical and molecular events involved in cell growth and differentiation. Often, in the case of serious skin injuries, skin burns, traumas, post-surgical injuries and in serious affections of the skin and of soft tissues, the repairing process is unable to reconstitute the integrity of the tissue and leads to results such as so-called "difficult" wounds, chronic ulcers, or hypertrophic scars and keloids, the latter being often associated with serious functional and aesthetic deficits.

In an effective wound healing process the dermis, i.e., the portion of skin below the epidermis, plays a central role in tissue regeneration and therefore in optimal repairing of the lesion. The dermis is the most voluminous part of the cutaneous organ and provides vascular support for survival of the epidermis. The cells of the dermis, and in particular fibroblasts, produce the connective matrix that is formed mainly by collagen fibers, which are important tissue and vascular growth factors and are the main players in the process of wound contraction.

Various substitutes of the dermis have been produced with the purpose of facilitating and increasing the processes of wounds healing. However, these substitutes have not proved themselves particularly effective in the treatment of so-called "difficult" wounds; these wounds are a very widespread pathology and destined to increase in the near future because of the progressive aging of the world population and due to the greater incidence of vascular problems in elderly individuals. Bad eating habits (and consequent metabolic disorders such as metabolic syndrome, diabetes, hyperdyslipidemias) and lifestyles (smoking, sedentariness) are also leading to an increase in the incidence of vascular problems (which are the first cause of this type of skin lesions) and not only in the elderly.

Currently, commercially available products are based on the use of dermal substitutes mainly without cells, constituted for example solely by matrices of collagen or hyaluronic acid. As an alternative, heterologous grafts of dermis from corpses or, when possible, of autologous skin taken from the patient himself are used. It is easy to understand that the use of autologous skin grafts is associated with the production of a further lesion at the site of the skin harvesting (which is not always feasible and/or convenient) and with the need for multiple, complex surgical procedures that imply a long and uncertain clinical recovery and are also very expensive. On the contrary, the use of heterologous skin may expose to the risk of immunological reaction and to potential risks of transmission of infectious diseases from the donor.

The curative effectiveness of the above mentioned dermal substitutes is therefore insufficient, indicating the need to optimize and investigate new strategies.

The aim of the present invention is to provide a dermal substitute that overcomes the drawbacks of the background art and actively promotes tissue regeneration.

This aim has been achieved by a dermal substitute that comprises a biocompatible matrix in which adipose tissue-derived mesenchymal stem cells (ADSC) and, optionally, precursors of adipose tissue-derived endothelial cells (VECs) are inserted.

The aim of the invention has also been achieved by the use of a biocompatible matrix in which adipose tissue-derived mesenchymal stem cells (ADSC) and, optionally, precursors of adipose tissue-derived endothelial cells (VECs) are inserted, for the preparation of a dermal substitute.

The aim of the invention has also been achieved by a dermal substitute comprising adipose tissue-derived SVF (Stromal Vascular Fraction), inserted in a biocompatible matrix, and by a method for its preparation.

The expression "adipose tissue-derived mesenchymal stem cells" (also known as "mesenchymal stem/stromal cells" or hereinafter even just "ADSC", from "Adipose Derived Stem/Stromal Cells") designates a population of cells derived from a cell fraction known as "Stromal Vascular Fraction" (also known as SVF) isolated from adipose tissue, advantageously subcutaneous adipose tissue.

The expression "biocompatible matrix" (hereinafter also just "matrix") designates a three-dimensional complex (hereinafter also termed scaffold) formed by biocompatible and, advantageously, reabsorbable biological material. Suitable matrices are for example matrices that comprise collagen and optionally glycosaminoglycans, for example collagen and chondroitin-6-sulfate and/or hyaluronic acid, or also matrices that comprise collagen and elastin. Such matrices are commercially available. One example of suitable matrix according to the invention, based on collagen and chondroitin-6-sulfate, is the product currently marketed under the trademark "Integra"; another example of matrix, based on collagen and elastin, is currently marketed under the trademark "Matriderm®". Other matrices that can be generally used are those that also comprise hyaluronic acid.

ADSC are multipotent cells that are capable of differentiating into different cell lines following suitable differentiating stimuli. Indeed it is known that ADSC are capable of differentiating into cells of the adipogenic, osteogenic, chondrogenic or also connective type, depending on the stimuli to which they undergo.

ADSC, when grown in a suitable culture medium, can have a morphology and an activity that are very similar to the cells that mainly constitute the dermis, that is fibroblasts.

In other words, ADSC, i.e., adipose tissue-derived mesenchymal stem cells, can be used to replace a fundamental part of the structure of the skin, that is the dermis.

Thus, by including ADSC in a biocompatible and reabsorbable matrix, it is possible to obtain a dermal (and/or hypodermic) substitute in which the cells produce extracellular substances (in the same way as fibroblasts of the dermis do) rich in collagen fibers and glycosaminoglycans produced ex novo, capable of facilitating skin regeneration, even where the severity and depth of the wound would not be treatable only by application of known dermal substitutes.

In one aspect of the invention, precursors of endothelial cells of adipose origin (VECs) are also inserted in said biocompatible matrix.

As an alternative, it is possible to include ADSC in the chosen biocompatible and reabsorbable matrix, in which VECs can also be inserted, and differentiate them with suitable growth stimuli.

According to another of its aspects, the invention relates to a dermal substitute that comprises ADSC inserted in a biocompatible matrix, as defined above.

According to another of its aspects, the invention relates to a dermal substitute that comprises ADSC and, optionally, VECs inserted in a biocompatible matrix, as defined above.

Preferably, in said dermal substitute the ADSC form an extracellular matrix.

Preferably, in said dermal substitute the ADSC and, optionally, the VECs are in culture in the presence of ascorbic acid as well.

In order to facilitate the growth of the ADSC, it is possible for example to grow them in cultures that contain one or more fibroblast growth factors.

The expression "fibroblast growth factor" designates one of the mammalian fibroblast growth factors, preferably one of the human fibroblast growth factors, for example the "basic" fibroblast growth factor or bFGF.

The dermal substitute according to the invention has a higher effectiveness if the ADSC inserted in it produce an "extracellular substance", the expression "extracellular substance" designating a mixture of components, generally in the form of gel, for example mainly constituted by polysaccharides (glycosaminoglycans) rich in fibrous proteins (particularly collagen fibers). In order to facilitate the production of extracellular substance, as defined above, by the ADSC, it is preferable to grow said cells in cultures that contain ascorbic acid, preferably at a concentration of approximately 250 $\mu$M. It is possible, by way of example, to add ascorbic acid to the culture medium of the ADSC used for seeding in the matrix.

Preferably, in the dermal substitute according to the invention ADSC and VECs are derived from subcutaneous adipose tissue.

Preferably, in the dermal substitute according to the invention said matrix is three-dimensional.

Preferably, said matrix comprises collagen.

Preferably, said matrix also comprises elastin and/or glycosaminoglycans and more preferably has a porosity between 70 and 100 $\mu$m.

In a preferred embodiment, the matrix of the dermal substitute according to the invention comprises collagen and chondroitin-6-sulfate. In a preferred embodiment, the matrix of the dermal substitute according to the invention does not comprise chitosan or partially demineralized spongy equine bone tissue. It has been found that by using the matrix that comprises collagen and chondroitin-6-sulfate, the cells penetrate into the scaffold and multiply, while the ones seeded on the other scaffolds remain on the surface without penetrating into the matrix.

According to another aspect, the invention relates to the dermal substitute according to the invention, as defined above, for its use in the treatment of wounds and to facilitate skin regrowth.

The term "wounds" designates herein any lesion of the skin, also, and above all, lesions extended to the dermis and/or also to the hypodermis.

According to a preferred aspect, the dermal substitute according to the invention is for human and/or veterinary use.

By way of non-limiting example, the dermal substitute according to the invention can be used in the treatment of even very deep wounds, such as acute or chronic wounds, including diabetic ulcers, vascular ulcers, skin bums, decubitus ulcers, traumatic lesions, post-surgery wounds, pressure sores or complications associated with wounds such as hypertrophic scars, keloids et cetera. Preferably, the dermal substitute according to the invention in combination with one or more further dermal substitutes is used for the treatment of wounds and for skin regeneration.

As mentioned above, the ADSC usable for the use according to the invention can derive, preferably, from subcutaneous adipose tissue, for example by micro-aspiration of a small quantity of subcutaneous adipose tissue. It is understood, therefore, that the technical solution proposed by the invention is of great clinical interest, because it uses a raw material that is easily accessible, can be harvested in day-hospital conditions, i.e., even in operating conditions that are not necessarily surgical, as occurs instead in the case of marrow harvesting or of explantation of skin flaps for autologous (or heterologous) transplantation.

The use of the dermal substitute according to the invention thus provides considerable progress in the field of wound healing, reducing times and providing a better aesthetic and functional result than the products of the background art. In particular, the dermal substitute according to the invention provides less scar tissue and a greater tissue "volume" where necessary (i.e., when the depth of the wound has caused an abundant loss of dermis and hypodermis), a lower "retraction" of the wound so as to result in a more acceptable aesthetic effect, an important fact mainly, but not only, in the case of bums or other deep wounds.

In practice, according to one of the embodiments of the invention, for the preparation of the dermal substitute according to the invention, a small quantity of adipose tissue, preferably subcutaneous, is harvested. The cells that belong to the SVF (Stromal Vascular Fraction) are extracted from this tissue by enzyme digestion of the connective stroma. The resulting ADSC are grown in the presence of a fibroblast growth factor (for example bFGF), expanded in vitro and seeded in a suitable scaffold, as defined above. Subsequently application in the seat of the lesion is performed. Further technical details are provided in the experimental section of the present description.

As an alternative to the use of ADSC, it is possible to use SFV as defined above, as obtained from extraction from adipose tissue, which is seeded in order to seed directly a suitable scaffold, as defined above; the system can be maintained for a brief period of time, for example for a few hours, in the presence of a suitable culture medium, and then application in the seat of the lesion is performed.

Thus, according to one of its aspects, the invention relates to a dermal substitute that comprises adipose tissue-derived SVF (Stromal Vascular Fraction), inserted in a biocompatible matrix, and optionally also adipose tissue-derived VECs.

According to a further aspect, the invention comprises the use of adipose tissue-derived SVF (Stromal Vascular Fraction), inserted in a biocompatible matrix, in which adipose tissue-derived VECs are also optionally inserted, in order to prepare a dermal substitute.

Technical details related to the preparation of the cells and of the dermal substitute according to the invention are provided in the experimental section that follows.

According to a preferred embodiment, the dermal substitute according to the invention comprises

- autologous multipotent stem/stromal cells obtained by micro-aspiration of subcutaneous adipose tissue (ADSC), which are fibroblast-like and capable of producing a connective matrix and cytokines which stimulate the regenerative and vascularization processes, like the dermal fibroblasts do;
- said cells being inserted in a three-dimensional biocompatible matrix that promotes integration in the host, facilitating the physiological distribution in the space of the cells seeded therein, guiding the migration of the host cells and facilitating the penetration of the newly formed vessels in the tissue.

The quantity of ADSC seeded in the biocompatible matrix can vary from $3 \times 10^4$ to $5 \times 10^4$ cells per $mm^3$. In general, and merely by way of example, it is possible to use $4 \times 10^4$ cells per $cm^3$ of matrix. However, different quantities can be used if desired or necessary.

The cells are inserted in the matrix by impregnation of the matrix itself with a liquid culture medium that contains said cells, preferably a complete culture medium (as described in the experimental section that follows).

According to another of its aspects, the invention relates to a method for the preparation of a dermal substitute that comprises:

    a. extracting the SVF from adipose tissue, preferably subcutaneous tissue;
    b. isolating the ADSC in culture;
    c. seeding the ADSC in a biocompatible matrix, as defined above; and
    d. stimulating the differentiation of the ADSC into dermal tissue, preferably in the presence of ascorbic acid.

According to another of its aspects, the invention relates to a method for preparing a dermal substitute that comprises:

    a. extracting SVF (Stromal Vascular Fraction) from adipose tissue, preferably subcutaneous tissue;
    b. isolating adipose tissue-derived mesenchymal stem cells (ADSC) in culture;
    c. isolating precursors of endothelial cells (VECs) in culture;
    d. seeding said ADSC and VECs in a biocompatible matrix at the air-culture medium interface; and
    e. stimulating the differentiation of said ADSC into dermal tissue and the vasculogenesis from said VECs.

According to another of its aspects, the invention relates to a method for preparing a dermal substitute that comprises:

    e. extracting the SVF from adipose tissue, preferably subcutaneous tissue;
    f. seeding the SVF in a biocompatible matrix, as defined above.

According to another of its aspects, the invention relates to a method for preparing a dermal substitute that comprises:

> a. extracting SVF from adipose tissue;
> b. seeding directly and without any intermediate culturing passages the SVF obtained in step a. in a biocompatible matrix.

Illustrative examples of the methods according to the invention are disclosed in the experimental section that follows.

The dermal substitute prepared according to the method disclosed herein constitutes a further object of the invention, and so does its use in the treatment of wounds.

The dermal substitute according to the invention has the form of a "sheet" or lamina, can be handled easily and can be applied easily on injured parts, to which it can be fixed by means, for example, of surgical staples placed by a stapler.

In addition to the advantages described above, the presence of the matrix makes the product even easier to handle and easier to apply compared to cell cultures alone (for example, simple epithelial cultures).

If desired or needed it is possible to apply a different epidermal substitute over the dermal substitute according to the invention. By way of illustration, after the dermal substitute according to the invention has been applied on the injured part (wound, ulcer, bum, et cetera), it is possible to arrange for example an epithelial lamina. This further epidermal substitute may be applied on the dermal substitute according to the invention, immediately or after some time.

Furthermore, it is possible, according to another embodiment of the invention, to seed in the matrix, simultaneously with the ADSC, also other cells such as fibroblasts, endothelial cells, epithelial stem cells (such as keratinocyte stem cells, urothelial stem cells, epithelial stem cells of the buccal mucosa) or even neuronal precursors.

According to this embodiment of the invention it is possible to create multi-tissue units (composed of multiple tissues of different origin) that can be used to obtain dermoepidermal substitutes (of skin) or even substitutes of buccal mucosa or urothelial mucosa. The neuronal precursors and endothelial cells can facilitate the formation of vessels and nerve fibers in these tissues.

Thus, if for example epithelial cells are seeded (such as keratinocytes) in the matrix simultaneously with the seeding with ADSC, the dermoepidermal substitute obtained from the seeding of keratinocytes and autologous ADSC in the three-dimensional matrix is applied to the seat of the lesion.

According to a further aspect of the invention, the ADSC, alone or with VECs, can also be used in association with other cell types such as epithelial cells (for example keratinocytes), endothelial cells and nerve cells in order to produce structures similar to organs (such as skin and mucosae).

As an alternative and according to a further aspect, the invention relates to a method for the treatment of wounds and for skin regrowth that comprises the application on the injured part (wound, ulcer, skin bum et cetera) of the dermal substitute according to the invention and subsequently (immediately after or some time after) of one or more different dermal substitutes adapted to regenerate the epithelium.

According to another of its aspects, the invention relates to the dermal substitute according to the invention in combination with one or more further epidermal substitutes for use in the treatment of wounds and for skin regrowth.

According to another of its aspects, the invention relates to a method for the treatment of wounds and for skin regrowth that comprises the application on the injured part (wound, ulcer, skin bum et cetera) of the dermal substitute according to the invention.

If desired or necessary, it is possible to modify genetically the ADSC before seeding them in the matrix, for example by insertion of genes for the production of trophic factors, thus improving the healing of the wounds or tissue damage.

Various assays have been performed in order to determine the characteristics of the ADSC and their suitability for the intended purposes of the invention. The details of said assays are provided in the experimental section.

In short, it has been observed that ADSC cells grow in vitro attached to the culture surface and their morphology in culture keeps a constant appearance in the different extractions performed, despite the cells originate from patients of different age and sex.

The Colony Forming Unit fibroblast (or CFU-F) has allowed theevaluation of the capacity of the ADSC to form clones and therefore to self-renew in culture. Analysis of the karyotype of the ADSC, in different steps of growth in culture, has proved the absence of chromosomal anomalies in the cells.

Furthermore, it has been observed that once the ADSC have been seeded in the biocompatible matrix, they spread in a homogenous manner, assuming a morphology similar to that of the fibroblasts of the dermis. An accurate analysis of the cell population has highlighted the presence of cells in the mitotic phase, thus proving the fact that these cells inside the matrix maintain their replication capacity.

As mentioned, ADSC are multipotent cells capable of differentiating into various types of cells, for example into cells of the adipogenic, osteogenic, chondrogenic or also connective type, according to the stimuli to which they are subjected.

Thus, according to another of its aspects, the invention relates to the use of adipose tissue-derived mesenchymal stem cells (ADSC), inserted in a biocompatible matrix, for the preparation of tissue substitutes, for example of tissues chosen among adipose tissue, bone tissue, cartilage tissue and connective tissue.

The invention also relates to a method for preparing a tissue substitute that comprises:

> g. extracting the SVF from adipose tissue, preferably subcutaneous tissue;
> h. isolating the ADSC in culture;
> i. seeding the ADSC in a biocompatible matrix, as defined above; and
> j. stimulating the differentiation of the ADSC into the desired tissue.

Experimental section

Example 1

Extraction of the stromal vascular fraction (SVF) and isolation and culture in vitro of adipose tissue-derived mesenchymal stem cells (ADSC).

The cellular component known as SVF has been isolated from subcutaneous adipose tissue obtained by liposuction (performed with a 1.5-2-mm needle cannula connected to a syringe) or from surgical samples after abdominoplasty interventions in healthy patients of both sexes and aged between 25 and 50 years.

All the procedures described hereinafter occur in sterile conditions.

The surgical sample of adipose tissue that arrives from the operating room is immersed in a sterile solution of DMEM (Dulbecco's Modified Eagle's Medium) with the addition of 1% PSA (penicillin-streptomycin-amphotericin B). A quick washing with physiological solution is performed. The subcutaneous adipose tissue with the septa of connective tissue and the vessels comprised therein are dissected by means of sterile scissors and forceps. The samples are immersed in a solution that contains 0.75 mg/ml collagenase I in HBSS (Hank's balance solution), 20 mM Hepes and 1.5% BSA (Bovine Serum Albumin) (pH 7.1). The quantity of this solution is equal to the fat volume that is intended to process. A fine micro-dissection of the adipose lobes is then performed by means of sterile scissors and forceps. The samples immersed in the collagenase solution are then transferred into disposable and sterile containers made of plastic material with stir bars under agitation at 37°C, 5%$CO_2$.

For the adipose tissue obtained by liposuction one proceeds directly by immersion thereof in the solution that contains 0.75 mg/ml collagenase I in HBSS, 20 mM Hepes and 1.5% BSA (pH 7.1) in disposable and sterile containers made of plastic material with stir bars under agitation at 37°C, 5% $CO_2$. The quantity of this solution is equal to the fat volume that is intended to process.

In both cases, after 1 hour the product of digestion in collagenase is filtrated by means of a layer of sterile gauze in order to eliminate the unwanted fat and the connective septa that have remained even after digestion.

The suspension is then centrifuged for 5 minutes at 1500 rpm (524 RCF or Relative Centrifugal Force).

The supernatant and fat fraction from mature adipocytes is eliminated by suction.

The cellular pellet is resuspended in the complete culture medium (see composition below) and simultaneously the cell count (after erythrocyte lysis) is performed by vitality exclusion assay with Trypan Blue.

For this purpose, 400 $\mu$l of cellular suspension are drawn and centrifuged for 5 minutes at 3000 rpm (2095 RCF). The supernatant is aspirated and discarded and the pellet is resuspended in 400 $\mu$l of erythrocyte lysis buffer (0.82% $NH_4Cl$ and 0.1% $KHCO_3$) at room temperature for 5 minutes.

At this point the cells are counted under the microscope by means of a Burker camera and seeded (passage PO) in flasks at a density of 100,000 cells per $cm^2$ in the complete culture medium optimized for the growth of the ADSC.

Isolation and culture in vitro of ADSC

The ADSC contained in the SVF cell fraction are isolated for their characteristics of growth attached on a dish and are kept in culture in a complete culture medium composed of:

- Dulbecco's Modified Eagle Medium/Ham's F12 (with a 3:1 ratio)
- 20% fetal bovine serum (FBS)
- 1% penicillin/streptomycin/amphotericin B antibiotic
- 2% glutamine
- 10 ng/ml basic fibroblast growth factor (bFGF).

The cells are amplified by seeding at each passage after PO (P1, P2, P3 et cetera) in a culture dish 20,000 cell/cm$^2$.

For the seedings, one proceeds first with trypsinization for 5 minutes at 37°C, using a solution containing 0.05% trypsin -0.02% EDTA.

Once the cells have detached, the action of trypsin is blocked by means of a medium containing 10% fetal bovine serum and the cells are centrifuged at 1200 rpm (335 RCF) for 8 minutes.

The supernatant is then aspirated and the pellet is resuspended in the complete culture medium.

The ADSC can be preserved by freezing. In this case, after trypsinization and centrifugation they are counted and resuspended in a solution constituted by:

80% fetal bovine serum (FBS)
10% DMSO
10% complete culture medium.
Usually, 2 x 10$^6$ cells per cryotube (or vial) are frozen.

The cells in the cryotubes are progressively frozen.

Kept for 48 hours at -80°C and then for an indefinite time in liquid nitrogen at -190°C.

For thawing, one proceeds rapidly by resuspending the cells of a cryotube in 10 ml of culture medium containing 10% fetal bovine serum. Centrifuging is performed at 1200 rpm for 8 minutes. The supernatant is aspirated and the pellet is resuspended in the complete culture medium.

The cells of the SVF population, particularly the ADSC, grow in vitro attached to the culture surface. The morphology, which is evaluated by bright field microscopy, appears fibroblast-like and comparable with bone marrow-derived mesenchymal stem cells. The morphology of the ADSC in culture maintains a constant appearance in the different extractions performed despite the origin of the cells from patients that differ for age or sex.

Example 2

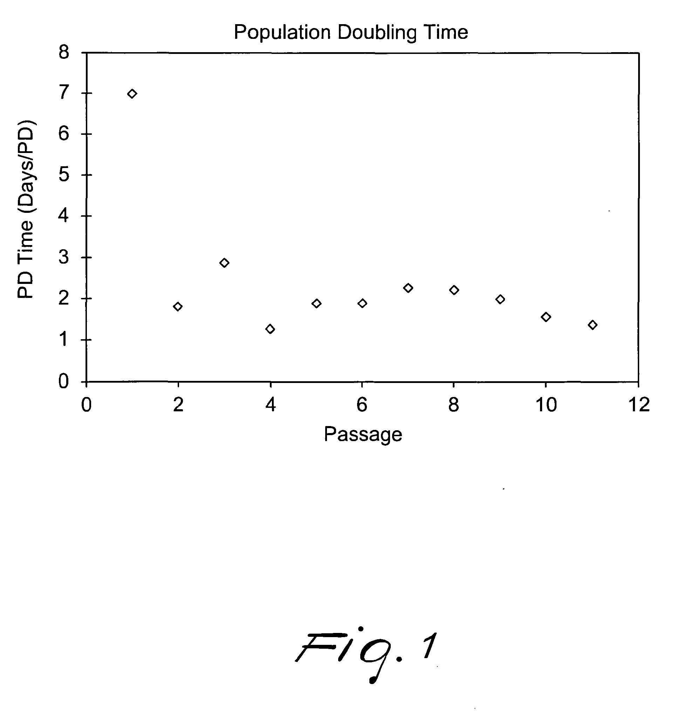

Cell proliferation test (Figure 1)

This test allows to evaluate the cell proliferation capacity of the cell population constituted by the ADSC and in particular the population doubling time (PDT). The proliferation values were obtained based on the calculation of Population Doubling Time (PDT) or proliferation time of the cell population and of Population Doubling (PD) i.e., the number of doublings in time. In particular, as reported in the literature:

$$PD=$$

$$PD = \log_2(N_i/N_0)$$

$$PDT = T_i/\log_2(N_i/N_0)$$

where $N_i$ is the number of cells at time i

No is the number of cells at time 0
$T_i$ is the time for which the cells have been in culture

The doubling time of the ADSC was found to be approximately 1.9 days.

Figure 1 shows the result of the cell proliferation test performed for various passages in culture.

Example 3

ADSC characterization

Example 3a

CFU-F assay

This assay was carried out to evaluate the capacity of the ADSC to form clones and therefore to self-renew in culture.

The test was performed for ADSC obtained from patients of both sexes, aged between 25 and 50 years and at different passages of culture. In particular, ADSC in passages P0, P1, P2 and P3 were considered, where P0 represents the passage at time 0, i.e., after extraction from adipose tissue, P1 represents cells in culture for one passage after extraction, P2 represents cells in culture for two passages after extraction and P3 represents cells in culture for three passages after extraction.

1 multiwell with 96 wells was seeded using the method of serial dilutions. 9 days after seeding the multiwell was washed with PBS buffer, fixed with formalin buffered at 4%, colored for 20 minutes with a 0.1% solution of toluidine blue. For clone counting, only cell groups containing at least 20 cells were considered as colonies. The positive wells are thus those that contain at least one colony. Then Poisson's equation is applied.

$$F_0 = e^{-u}$$

where $F_0$ is the fraction of negative wells without colonies, u is the average number of colonies per well and e is the base of the natural logarithm (or Napier's number).

When u is equal to 1:

$$F_0 = e^{-u} = e_{-1} = 1/e = 1/2.71 = 0.37 = 37\%$$

In this way, it is assumed that the column that contains at least three negative wells out of eight (37%) is the one that allows to identify the cell concentration that is necessary to obtain a single CFU.

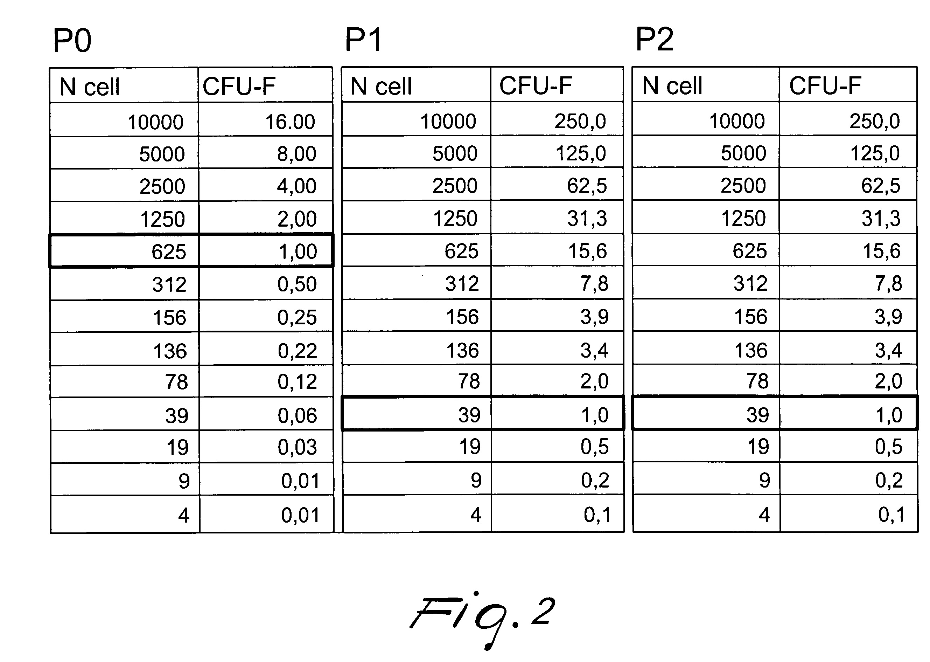

Figure 2 shows the result of CFU-f performed with the method of serial dilutions. The results point out that while at P0 only 1:625 seeded cells showed the capacity to form clones, at P1 and P2 as many as 1:39 seeded cells showed the capacity to form clones. This result shows that the culture method selected cells with clonogenic capacities.

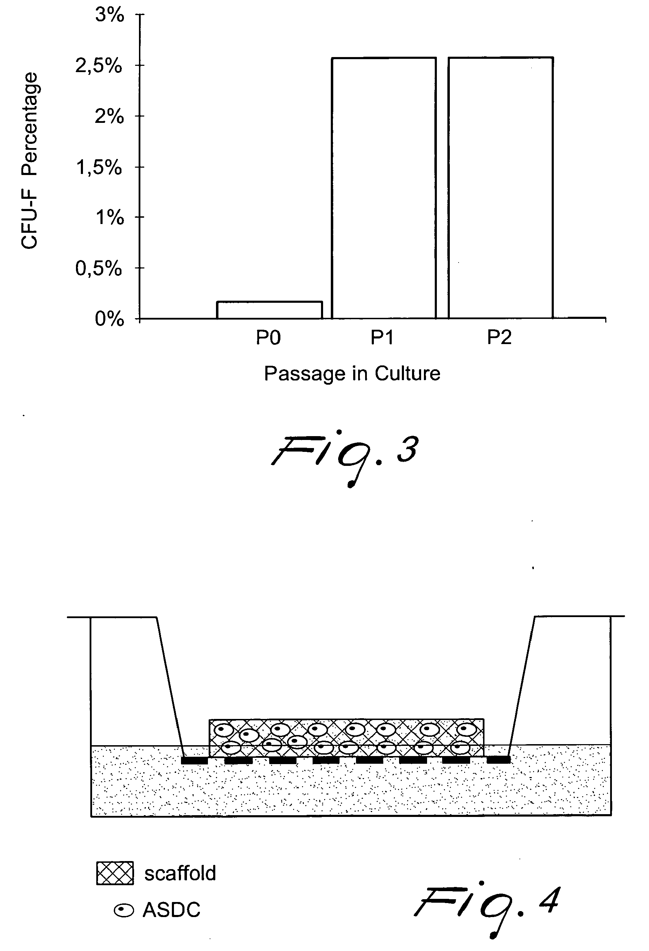

Another method for evaluating CFU-f consists in seeding ADSC at low density on dishes. In particular, the dishes are seeded with ADSC at different passages (P0, P1, P2...) and with low and progressively increasing densities: 5, 25, 95, 238, 500 and 1000 cells/cm$^2$. In this case also, the test was performed for ADSC obtained from patients of both sexes, aged between 25 and 50 years and at different passages (Figure 3).

CFU-f allowed to demonstrate the capacity of the ADSC to form clones and therefore to self-renew in culture.

Example 3b

Immunophenotype analysis by flow cytometry (FACS)

The ADSC are subjected to immunophenotype analysis by means of a cytofluorimeter in order to verify the presence of ADSC markers recognized by the international literature.

The ADSC are treated with different antibodies for 30 minutes at + 4°C.

The results are then read, and they will indicate the presence or not of the antigens on the ADSC, by means of a cell sorter.

The test was performed, in this case also, for ADSC obtained from patients of both sexes, aged between 25 and 50 years and at different passages.

Furthermore, analysis by FACS was also performed in parallel on normal human fibroblasts with the purpose of making a comparison.

Immunophenotype analysis shows that ADSC have a immunophenotype profile that is very similar to the one observed in bone marrow-derived mesenchymal stem cells (hBM-MSC), because they are positive to mesenchymal markers such as CD73, CD90, CD105, CD146, and negative for hematopoietic markers (CD45).

Example 3c

Comparison with human fibroblasts

Normal human fibroblasts, cells of mesenchymal origin that are responsible for the formation of the matrix of connective tissue and represent the main cell type present in the dermis, were used as cell population with which to compare the ADSC. The fibroblasts were obtained from healthy skin harvested by punch biopsy. The piece, placed in a sterile Petri dish, is treated according to the method originally described by Rheinwald and Green.

Briefly, the adipose tissue is eliminated from the skin by using sterile forceps and scissors. The piece is then cut into smaller fragments and disinfected by means of two passes in 70% alcohol and two passes in PBS (Phosphate Buffered Saline) buffer. The fragments were then immersed in DMEM medium, with the addition of PSA (1%), and subsequently placed in refrigerator at 4°C for at least 2 hours (in order to reduce any possible bacterial charge). Subsequently, the tissue was transferred in Dispase (collagenase IV) for the whole night at 4°C, in order to separate the epidermis from the dermis at the level of the lamina lucida. Once separation was performed, also by using sterile forcipes, the dermis was further fragmented into small pieces, which were subsequently transferred in an F25 flask (25 cm 2) with 1 ml of DMEM culture medium with the addition of 10% fetal bovine serum, 4 mM glutamine, 50 ID/ml PSA, so that the fragments of dermis could remain well attached to the plastic. The flask is kept initially for three days in incubator without changing the medium, which was subsequently changed every 48 hours. The fragments of dermis are not taken from the flask until the fibroblasts begin to expand from the dermis, one week after seeding.

The ADSC cultures in the scaffold proved themselves morphologically and functionally (capacity to produce extracellular substance, as defined herein) similar to human fibroblasts.

Example 4

Karyotype analysis

In order to prove the absence of transformation of the ADSC in culture, karyotype analysis was performed at different passages. After trypsinization and centrifugation, the ADSC in suspension are stimulated to mitosis by phytohemagglutinin (PHA) and halted in the mitotic metaphase with colchicine. The cells are subjected to osmotic lysis in a suitable buffer solution and the metaphase plates are isolated. The chromosomes are then stained with the banding method. The metaphase plates are finally photographed. Karyotype analysis at different passages of the ADSC proved the absence of chromosome anomalies of these cells after various passages in culture (passage 1 and passage 3).

Example 5

Preparation of a dermal substitute according to the invention

The matrix (or scaffold) used is a system constituted by a porous three-dimensional matrix of biological material (for example collagen fibers) that can be reabsorbed completely over time. The matrix must ensure the replication and growth of the ADSC seeded in it, must be constituted by a material that is sufficiently rigid and resistant so as to be easily transported and applied in the graft site and must not cause inflammatory reactions by the host. A suitable scaffold is, for example, the one currently marketed with the name INTEGRA®. This scaffold is constituted by a porous matrix composed of reticulated collagen fibers, obtained from bovine tendon, and by a glycosaminoglycan (such as chondroitin-6-sulfate), produced with controlled porosity and defined degradation rate. The matrix used must be sterile and possibly kept in a phosphate buffer at +2°C.

The method for the production of cellularized dermal substitute entails the seeding of the ADSC inside the scaffold and keeping the cellularized scaffold at the air-culture medium interface (for example on multiwell dishes with 6 wells of the Transwell type). For this purpose, the scaffold is rested on a grid-like support and the cells are kept in direct contact with the atmosphere (see Figure 4). In this model, the morphologic and architectural characteristics of the dermis that will develop can be overlapped on those of in vivo dermis. In particular, the ADSC will arrange themselves in the porous structure of the scaffold mimicking a three-dimensionality function that is essential for adequate tissue reparation. The dermal substitute is kept in the complete culture medium, substituted every other day, for a period of 3 weeks at 37°C, 5%$CO_2$. Before seeding the ADSC inside the scaffold, the matrix is cut and arranged inside a Petri capsule with physiological solution for 15 minutes. Then it is moved into a new Petri capsule that contains a solution of Hank's Balance Solution and 1% of PSA for 1 hour at 37°C, 5% $CO_2$.

The method for seeding the ADSC inside the matrix entails that after trypsinization and centrifugation, the ADSC at passage P1 or P2 are counted and seeded in number of 400,000/$cm^2$, resuspended in complete culture medium. This cell suspension is then distributed on the surface of the scaffold, dried on sterile gauze and arranged on the surface of the grid for emerged growth (for example Transwell surface).

After three weeks, the cellularized dermal substitute can be studied by histology and immunohistochemistry. For the histological preparations, the samples are fixed in formalin buffered at 4%, included in paraffin and cut with a microtome. In the case of preparations for immunohistochemistry, the samples are frozen and subsequently cut with a cryostat to a thickness of 4 $\mu$m.

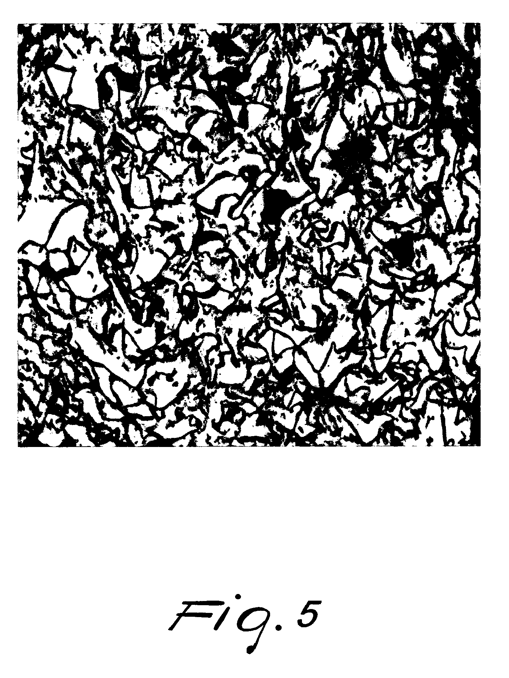

The sections are stained with hematoxylin-eosin in order to highlight the morphology of the tissue (Figure 5). In the histological preparations, a homogeneous distribution of the cells inside the scaffold, with presence of mitotic figures, a cell replication indicator, is observed.

The sections are also used for immunohistochemical analysis of the main proteins that constitute the extracellular matrix of the connective tissue by means of anti-collagen I antibodies, collagen III and fibronectin of human origin. The immunohistochemical investigation highlights the presence of collagen fibers and fibronectin of human origin newly deposited among the collagen fibers of bovine origin that constitute the scaffold.

Example 6

Check of increased production of connective matrix by addition of ascorbic acid

The cellularized scaffold was kept in culture with the following medium containing
Dulbecco's Modified Eagle Medium/Ham's F12 (with 3:1 ratio)
20% fetal bovine serum (FBS)
1% penicillin/streptomycin/amphotericin antibiotic
2% glutamine
10 ng/ml basic fibroblast growth factor (bFGF)
250 $\mu$M ascorbic acid.
The cells are kept in culture for 30 days at 37°C, 5%$CO_2$. The quantity of extracellular matrix produced can be studied by histology (hematoxylin-eosin; Masson's trichrome and Alcian Blue) and immunohistochemistry (with human anti-collagen I, human anti-collagen III and human anti-fibronectin antibodies). The results of the assay showed that there is an increase in the production of connective matrix in presence of ascorbic acid.

Example 7

Treatment of the cellularized scaffold with differentiating factors to obtain bone tissue

The cellularized scaffold was treated with a specific differentiating culture medium capable of inducing the differentiation of stem/stromal/ mesenchymal cells in osteoblasts.
The cells were seeded in the scaffold as described previously at a concentration of 40,000 cells/$cm^2$. Then an osteogenic differentiation treatment was performed by means of differentiating medium (composed of DMEM/F12, 10% FBS, 1% PSA, 10 $\mu$M p-glycerophosphate, 10 nM Dexamethasone, 0.25 mM Ascorbic Acid) for 21 days.
Differentiation in the osteogenic line was proved by demonstration of alkaline phosphatase activity and by specific staining such as Alizarin Red.

Example 8

Evaluation of the capacity of SVFs to differentiate into endothelial vascular cells

In order to evaluate whether SVFs can develop in vitro the characteristics of vascular endothelial cells (VECs), after passage P0, previously described, the SVFs were grown in a medium (EBM-2, Cambrex) optimized for the growth of vascular endothelial cells containing:

- 2% FBS,
- EGM-2 Bullet Kit,
- 10 ng/ml bFGF,
- 0.5 ng/ml VEGF,
- 22.5 $\mu$g/ml heparin,
- 20 ng/ml IGF-1,
- 5 ng/ml EGF,
- 0.2 $\mu$g/ml hydrocortisone,
- 1 $\mu$g/ml ascorbic acid.

The SVFs are seeded at a density of 20,000 cells/$cm^2$ and cultured with this medium for 10 days; the medium is changed every 3 days.
The SVFs are generally cultured with a medium that contains Dulbecco's Modified Eagle Medium/Ham's F12 (DMEM/F12 with 3:1 ratio), 20% fetal bovine serum (FBS), 1% penicillin/streptomycin/amphotericin B antibiotic, 2% glutamine, 10 ng/ml bFGF.
In one experiment, the SVFs were cultured with the EGM2 medium (medium commercially available for the growth of vascular endothelial cells). It has been observed that cells cultured with this medium reach confluence faster and appear to be more compact with larger nuclei than the cells cultured in DMEM. A cell proliferation assay enables to confirm this data item observed only morphologically.

In order to evaluate whether SVFs cultured in EGM2 medium express endothelial markers, the cells were seeded in coverslip inside multiwell dishes with 6 wells and were differentiated with EGM2. 10 days later the cells are washed with PBS and fixed with methanol for 5 minutes. The cells are washed again with PBS and treated with a permeabilizing agent, Triton X100, 0.05% for 8 minutes. After a further washing in PBS the cells are incubated with 5% of serum for 1 hour and then incubated with the following antibodies for 1 hour:

- CD31

- CD34

- CD45

The cells are then incubated with the fluorescent secondary antibody while the nuclei are stained with DAPI. The cells are observed with a fluorescence microscope.

It is observed that endothelial markers CD31, CD34 are positive, and negative for CD45, proving that SVF cells cultured after 10 days with EGM2 differentiate and assume the characteristics of vascular endothelial cells (VECs). The nuclei are stained with DAPI.

- Provision of cellularized dermal substitute by means of co-culture.

In order to evaluate the formation of a cellularized dermal substitute, a co-culture system (two different cell types cultured together) was used:

- ADSC cultured with EGM2 for 10 days (becoming VECs)
- ADSC cultured with Dulbecco's Modified Eagle Medium/Ham's F12 (DMEM/F12 with 3:1 ratio), 20% fetal bovine serum (FBS), 1% penicillin/streptomycin/amphotericin B antibiotic, 2% glutamine, 10 ng/ml bFGF for 10 days.

The VECs are seeded (400,000 cells/cm$^2$) first inside the scaffold, a biocompatible matrix, at the air-culture medium interface and are kept in culture with EGM2 medium for a further 3 days.

After three days of culture, the ADSC (400,000 cells/cm$^2$), cultured previously with a medium containing Dulbecco's Modified Eagle Medium/ Ham's F12 (DMEM/F12 with 3:1 ratio), 20% fetal bovine serum (FBS), 1% penicillin/streptomycin/amphotericin B antibiotic, 2% glutamine, 10 ng/ml bFGF, are added inside the same scaffold in which the VECs had been seeded.

At this point the cells (ADSC and VECs) are kept in culture together inside the scaffold for three weeks in a medium containing Dulbecco's Modified Eagle Medium/Ham's F12 (DMEM/F12 with 3:1 ratio), 20% fetal bovine serum (FBS), 1% penicillin/streptomycin/amphotericin B antibiotic, 2% glutamine, 10 ng/ml bFGF with the addition of 250 $\mu$M ascorbic acid.

The disclosures in Italian Patent Application no. MI2010A000601 from which this application claims priority, are incorporated herein by reference.

**Claims**

1. A dermal substitute comprising adipose tissue-derived mesenchymal stem cells (ADSC) inserted in a biocompatible matrix.

2. The dermal substitute according to claim 1, wherein precursors of adipose tissue-derived endothelial cells (VECs) have been further inserted in the biocompatible matrix.

3. The dermal substitute according to claim 1 or 2, wherein said mesenchymal stem cells form an extracellular matrix.

4. The dermal substitute according to claims 1-3, wherein said mesenchymal stem cells and said precursors of endothelial cells (VECs), if present, are kept in culture in the presence also of ascorbic acid.

5. The dermal substitute according to claims 1-4, wherein said adipose tissue is subcutaneous adipose tissue.

6. The dermal substitute according to claims 1 to 5, wherein said matrix is three-dimensional.

7. The dermal substitute according to claims 1 to 6, wherein said matrix comprises collagen.

8. The dermal substitute according to claims 1 to 7, wherein said matrix also comprises elastin and/or glycosaminoglycans.

9. The dermal substitute according to claim 8, wherein the matrix has a porosity between 70 and 100 $\mu$m.

10. The dermal substitute according to claims 1 to 9, wherein said matrix comprises collagen and chondroitin-6-sulfate.

11. The dermal substitute according to claims 1 to 10, wherein said matrix does not comprise chitosan or partially demineralized spongy equine bone tissue.

12. The dermal substitute according to claims 1 to 11, further comprising other types of cells.

13. The dermal substitute according to claims 1 to 12, for use in the treatment and reparation of wounds and for facilitating skin regeneration.

14. The dermal substitute according to claims 1 to 13, for use in the treatment of acute and chronic wounds, diabetic ulcers, vascular ulcers, skin burns, bedsores, traumatic injuries, postoperative wounds, pressure sores, complications of wounds, hypertrophic scars and keloids.

15. The dermal substitute according to claims 1 to 14, in combination with one or more further dermoepidermal substitutes, for use in the treatment of wounds and skin regeneration.

16. Use of a biocompatible matrix in which adipose tissue-derived mesenchymal stem cells and optionally also precursors of adipose tissue-derived endothelial cells (VECs) are inserted, both for the preparation of a dermal substitute.

17. A dermal substitute comprising adipose tissue-derived SVF (Stromal Vascular Fraction), inserted in a biocompatible matrix.

18. A method for the preparation of a dermal substitute comprising:

    a. extracting SVF (Stromal Vascular Fraction) from adipose tissue, preferably subcutaneous tissue;
    b. isolating adipose tissue-derived mesenchymal stem cells (ADSC) in culture;
    c. seeding said ADSC in a biocompatible matrix; and
    d. stimulating the differentiation of said ADSC into dermal tissue.

19. A method for the preparation of a dermal substitute comprising:

    a. extracting SVF (Stromal Vascular Fraction) from adipose tissue, preferably subcutaneous tissue;
    b. isolating adipose tissue-derived mesenchymal stem cells (ADSC) in culture;
    c. isolating precursors of endothelial cells (VECs) in culture;
    d. seeding said ADSC and VECs in a biocompatible matrix at the air-culture medium interface;
    e. stimulating the differentiation of said ADSC into dermal tissue and vasculogenesis from said VECs.

20. A method for the preparation of a dermal substitute that comprises:

    a. extracting SVF from adipose tissue;
    b. seeding directly and without any intermediate culture passages the SVF obtained in step a. in a biocompatible matrix.

Fig. 1

## P0

| N cell | CFU-F |
|---:|---:|
| 10000 | 16.00 |
| 5000 | 8,00 |
| 2500 | 4,00 |
| 1250 | 2,00 |
| 625 | 1,00 |
| 312 | 0,50 |
| 156 | 0,25 |
| 136 | 0,22 |
| 78 | 0,12 |
| 39 | 0,06 |
| 19 | 0,03 |
| 9 | 0,01 |
| 4 | 0,01 |

## P1

| N cell | CFU-F |
|---:|---:|
| 10000 | 250,0 |
| 5000 | 125,0 |
| 2500 | 62,5 |
| 1250 | 31,3 |
| 625 | 15,6 |
| 312 | 7,8 |
| 156 | 3,9 |
| 136 | 3,4 |
| 78 | 2,0 |
| 39 | 1,0 |
| 19 | 0,5 |
| 9 | 0,2 |
| 4 | 0,1 |

## P2

| N cell | CFU-F |
|---:|---:|
| 10000 | 250,0 |
| 5000 | 125,0 |
| 2500 | 62,5 |
| 1250 | 31,3 |
| 625 | 15,6 |
| 312 | 7,8 |
| 156 | 3,9 |
| 136 | 3,4 |
| 78 | 2,0 |
| 39 | 1,0 |
| 19 | 0,5 |
| 9 | 0,2 |
| 4 | 0,1 |

*Fig.2*

EP 2 374 485 A1

Fig. 3

⊠ scaffold
⊙ ASDC

Fig. 4

Fig. 5

Europäisches
Patentamt

European
Patent Office

Office européen
des brevets

# EUROPEAN SEARCH REPORT

Application Number

EP 11 42 5098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TROTTIER VALERIE ET AL: "IFATS Collection: Using Human Adipose-Derived Stem/Stromal Cells for the Production of New Skin Substitutes", STEM CELLS (MIAMISBURG), vol. 26, no. 10, 2008, pages 2713-2723, XP002604955, ISSN: 1066-5099 | 1-5,8, 12-15 | INV. A61L27/60 A61Q19/00 A61L27/38 |
| Y | * page 2714, column 1, paragraphs 2,3 * <br> * page 2714, column 2, paragraph 2 - paragraph 4 * <br> * page 2715, column 1, paragraph 6 - column 2, paragraph 2 * <br> * page 2716, column 2, paragraph 2; figure 1 * <br> * page 2719, column 2 - page 2720, column 1, paragraph 1 * | 7 | |
| X | WO 97/41208 A1 (UNIV CASE WESTERN RESERVE [US]; SORRELL J MICHAEL [US]; CAPLAN ARNOLD) 6 November 1997 (1997-11-06) | 1,4-6, 8-13 | |
| Y | * page 5, paragraphs 1,2 * <br> * page 8, paragraph 1 * <br> * page 9, paragraph 1 * | 7 | **TECHNICAL FIELDS SEARCHED (IPC)** <br> A61L <br> C12N <br> A61Q <br> A61K |
| X | HUH C H ET AL: "Effects of mesenchymal stem cells in the reconstruction of skin equivalents", JOURNAL OF DERMATOLOGICAL SCIENCE, ELSEVIER SCIENCE PUBLISHERS, SHANNON, IE, vol. 46, no. 3, 1 June 2007 (2007-06-01), pages 217-220, XP027006547, ISSN: 0923-1811 [retrieved on 2007-04-20] | 1,3,4,8, 12 | |
| Y | * page 217, column 1, paragraph 3 - column 2, paragraph 1 * <br> * page 218, column 2, paragraph 3 - page 219, column 1, paragraph 2 * | 7 | |

-/--

The present search report has been drawn up for all claims

| Place of search | Date of completion of the search | Examiner |
|---|---|---|
| Munich | 12 July 2011 | Bonello, Steve |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 11 42 5098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MA K ET AL: "Differentiation of bone marrow-derived mesenchymal stem cells into multi-layered epidermis-like cells in 3D organotypic coculture", BIOMATERIALS, ELSEVIER SCIENCE PUBLISHERS BV., BARKING, GB LNKD- DOI:10.1016/J.BIOMATERIALS.2009.02.025, vol. 30, no. 19, 1 July 2009 (2009-07-01), pages 3251-3258, XP026097991, ISSN: 0142-9612 [retrieved on 2009-03-13] | 1,2,4,5, 8,12 | |
| Y | * page 3251, column 2, paragraph 2 - page 3252, column 1, paragraph 1 * * page 3252, column 2, paragraph 4 * * page 3255, column 2, paragraph 4 * * page 3257, column 2, paragraphs 2,3 * ----- | 7 | |
| T | MADONNA ROSALINDA ET AL: "In vitro neovasculogenic potential of resident adipose tissue precursors", AMERICAN JOURNAL OF PHYSIOLOGY. CELL PHYSIOLOGY, AMERICAN PHYSIOLOGICAL SOCIETY, US, vol. 295, no. 5, 1 November 2008 (2008-11-01), pages C1271-C1280, XP002592954, ISSN: 0363-6143 [retrieved on 2009-09-11] * page C1271, column 2, paragraph 1 * * page C1278, column 1, paragraph 2 - column 2, paragraph 2 * ----- | 2 | TECHNICAL FIELDS SEARCHED (IPC) |

The present search report has been drawn up for all claims

| Place of search | Date of completion of the search | Examiner |
|---|---|---|
| Munich | 12 July 2011 | Bonello, Steve |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**                    EP 11 42 5098

This annex lists the patent family members relating to the patent documents cited in  the above-mentioned European search report.
The members are as contained in the European Patent Office EDP file on
The European Patent Office is in no way liable for these particulars which are merely given for the purpose of information.

12-07-2011

| Patent document cited in search report | | Publication date | Patent family member(s) | | Publication date |
|---|---|---|---|---|---|
| WO 9741208 | A1 | 06-11-1997 | AU | 2808397  A | 19-11-1997 |
| | | | CA | 2253724  A1 | 06-11-1997 |
| | | | EP | 0953040  A1 | 03-11-1999 |
| | | | JP | 2000508922  A | 18-07-2000 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• IT MI20100601 A